(19) Europäisches Patentamt
European Patent Office
Office européen des brevets

(11) **EP 4 173 601 B1**

(12) **EUROPEAN PATENT SPECIFICATION**

(45) Date of publication and mention
of the grant of the patent:
**26.11.2025 Bulletin 2025/48**

(21) Application number: **21939993.8**

(22) Date of filing: **29.06.2021**

(51) International Patent Classification (IPC):
*A61F 5/56* (2006.01)     *A61C 5/00* (2017.01)

(52) Cooperative Patent Classification (CPC):
**A61F 5/566**

(86) International application number:
**PCT/KR2021/008203**

(87) International publication number:
**WO 2022/270662 (29.12.2022 Gazette 2022/52)**

(54) **ORAL APPLIANCE**

ORALE VORRICHTUNG

APPAREIL BUCCAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2021 KR 20210082862**

(43) Date of publication of application:
**03.05.2023 Bulletin 2023/18**

(73) Proprietor: **The Sleep Factory Inc.**
**Incheon 22004 (KR)**

(72) Inventors:
• **PARK, Young Hyon**
**Incheon 22003 (KR)**
• **PARK, Jun Hyuk**
**Incheon 22003 (KR)**
• **PARK, Jun Young**
**Incheon 22003 (KR)**
• **PARK, Jun Won**
**Incheon 22003 (KR)**

(74) Representative: **Nederlandsch Octrooibureau**
**P.O. Box 29720**
**2502 LS The Hague (NL)**

(56) References cited:
JP-A- H10 174 697     KR-A- 20170 047 186
KR-B1- 101 828 841     KR-B1- 102 058 104
KR-B1- 102 159 365     KR-B1- 102 185 512

## Description

## BACKGROUND OF THE DISCLOSURE

Field of the disclosure

The present disclosure relates to an oral appliance, and more particularly, to an oral appliance for preventing snoring and sleep apnea and improving mouth breathing into nasal breathing.

Related Art

Snoring occurs when the air inhaled through the nose passes through the nasal cavity and flows through the uvula or the back of the tongue, and here, if this section is pressed or distorted by a surrounding tissue so the air enters in a state in which the airway is narrower than a normal state. In the deformed and narrowed airway, the flow of air is not smooth and is stagnant and surrounding soft tissues vibrate. Here, if the uvula shivers or shivering is spread to an empty space around the noise to resonate, noise occurs. Here, when the state is so severe that if the air flow itself stops in this section, sleep apnea occurs.

In order to prevent such snoring and sleep apnea, a positive air pressure device that forcibly supplies air to the airways during sleep may be used. However, although such a positive air pressure device is effective for snoring and sleep apnea, there are problems in that the device is inconvenient to use, difficult to adapt to the human body, and costly.

In addition, there is a surgical method to cut the soft tissue of the oropharynx, which, however, is rejected because of the problems that recurrence is common and there are side effects according to the operation.

In addition, there is an oral appliance which is mounted on the maxillary and mandibular dentition in the oral cavity to prevent snoring and sleep apnea. This oral appliance allows the tongue to be naturally pulled forward as the mandible moves forward, thereby relieving pressure acting on the airway by the tongue.

The oral appliance includes a maxilla and mandible integrated oral appliance in which the maxiliary teeth insertion portion, which is a portion into which the teeth included in the maxiliary dentition is inserted, and a mandibular teeth insertion portion, which is a portion into which the teeth included in the mandibular dentition is inserted, are integrated, and a maxilla and mandible separated oral appliance in which the maxiliary teeth insertion portion and the mandibular teeth insertion portion are separated.

As described above, the maxilla and mandible integrated oral appliance includes the integrated maxiliary teeth insertion portion and mandibular teeth insertion portion. Therefore, when the maxilla and mandible integrated oral appliance is used, the mandible cannot move in an advanced state and should be maintained in the corresponding state during sleep. This leads to a problem in that the mandibular joint or surrounding muscles or ligaments are stiff or painful.

In order to solve this problem, in the maxilla and mandible separated oral appliance, the maxiliary teeth insertion portion and the mandibular teeth insertion portion are separated as described above so that the mandible may move during sleep. In addition, the maxiliary teeth insertion portion and the mandibular teeth insertion portion are connected by a connection member so that the mandible may move. However, such a maxilla and mandible separated oral appliance cannot solve the problem mentioned above because movement of the mandible is constrained by the connection member traversing the maxiliary and mandibular parts laterally to connect the maxiliary and mandibular parts.

Therefore, the inventor of the present disclosure has attempted to solve the problems mentioned above by providing an oral appliance which allows the mandible to move naturally, even when worn, so that stiffness of muscles and ligaments may be alleviated in Korean Patent Registration No. 10-1463021.

However, even in this case, there is a limitation in that the temporomandibular joint is still stressed and the surrounding muscles or ligaments are stiff, causing pain to the user when worn for a long time. In addition, as the mandible moves forward, the tongue moves forward and protrudes, and the tongue protruding forward retreats backward during long-term sleep. As a result, the airway located at the back of the tongue is pressed, degrading the effect of alleviating snoring and sleep apnea.

In addition, when snoring and sleep apnea occur, an oxygen deficiency gradually worsens and the human body performs mouth breathing by opening the mouth to increase the insufficient amount of oxygen. When the mouth is open for mouth breathing, the mandible rotates backwards and downwards and the tongue associated with the mandible also loses contact with the roof of the mouth and is positioned at a lower portion, and the tongue lowered downwards gradually hangs back over time to press the airway to aggravate snoring and sleep apnea.

Therefore, the development of an appliance capable of alleviating snoring and sleep apnea by moving the mandible forward, while not putting strain on the temporomandibular joint and surrounding muscles and placing the tongue in the anterior region of the palate to change mouth breathing into nasal breathing, is necessary.

Patent document, published on 4 January 2021, discloses a snoring prevention device comprising: an upper jaw portion (10) into which the upper jaw is inserted; a lower jaw portion (20) into which the lower jaw is inserted; and a connection portion (30) which connects the upper and lower jaw portions (10, 20), wherein the upper jaw portion (10) is formed with an upper jaw tooth groove (11) so that upper jaw teeth are positioned, the lower jaw portion (20) is formed with a lower jaw tooth groove (11) so that lower jaw teeth are positioned, and an air passage hole (31) is formed in the connection portion (30).

## DETAILED DESCRIPTION

## TECHNICAL PROBLEM

The present disclosure provides an oral appliance which allows a mandible to move forward, without putting strain on the temporomandibular joint and surrounding muscles, and which can be worn for a long time during sleep, thereby improving snoring.

The present disclosure also provides an oral appliance which allows a tongue to move forward along with a mandible as well as expands a space in which the tongue is positioned so that the tongue is naturally placed at an anterior region of a palate to induce conversion of mouth breathing to nasal breathing, thereby improving snoring and sleep apnea.

## SUMMARY

In an aspect, an oral appliance detachably worn in an oral cavity, the oral appliance having a shape corresponding to a dentition in the oral cavity, includes: a first dentition accommodation portion formed in a shape corresponding to a dentition of a maxilla and accommodating the dentition of the maxilla; a second dentition accommodation portion formed in a shape corresponding to a dentition of a mandible and accommodating the dentition of the mandible; and a spacer connecting the first dentition accommodation portion and the second dentition accommodation portion and formed to have a predetermined thickness so that the dentition of the maxilla and the dentition of the mandible are spaced apart from each other by a predetermined interval or greater.

The first dentition accommodation portion, the second dentition accommodation portion, and the spacer may be integrally formed.

The dentition of the maxilla and the dentition of the mandible may be spaced apart from each other by a predetermined interval such that the maxilla and the mandible may be open by the predetermined thickness of the spacer, thereby expanding space in the oral cavity.

The thickness of the spacer may satisfy Equation 1 below.

$$[\text{Equation 1}]$$

$$\frac{T_1 + T_2}{6} \leq Ts \leq T_1 + T_2$$

wherein $T_1$ is a thickness of the first dentition accommodation portion, $T_2$ is a thickness of the second dentition accommodation portion, and $T_S$ is a thickness of the spacer.

The oral appliance may further comprises a tongue tip mounting portion as a space in which an inner wall surface adjacent to the oral cavity corresponding to incisors of the first dentition accommodation portion is open to allow the tip of the tongue to be mounted.

The tongue tip mounting portion may not have an inner wall surface adjacent to the oral cavity corresponding to the incisors of the first dentition accommodation portion so that the tip of the user's tongue is placed behind upper incisors when worn by the user.

The guide portion may be formed at both end portions of the first dentition accommodation portion guiding the user's teeth to be accommodated in the first dentition accommodation portion and the second dentition accommodation portion as the inner wall surface of the first dentition accommodation portion adjacent to the oral cavity extends by a predetermined distance.

The guide portion may be formed in a position corresponding to the dentition of a canine of the first dentition accommodation portion.

The guide portion may extends in a longitudinal direction along the dentition of the canine in the anterior region of the maxilla to further accommodate at least a portion of the premolar portion of the maxilla.

An inner wall of the second dentition accommodation portion adj acent to the oral cavity may be formed so that the user's teeth are fixed by frictional contact, and the first dentition accommodation portion may be formed to be spaced apart from the teeth of the maxilla by a predetermined distance in vertical and horizontal directions.

At least a portion of an inner wall between the first dentition accommodation portion and the second dentition accommodation portion may be open.

The inner wall between the first dentition accommodation portion and the second dentition accommodation portion may correspond to an inner wall of a portion corresponding to the incisors.

## ADVANTAGEOUS EFFECTS

The oral appliance of the present disclosure allows the mandible to move forward, without putting strain on the temporomandibular joint and surrounding muscles, so that the oral appliance may be worn for a long time during sleep, thereby improving snoring.

In addition, the oral appliance of the present disclosure allows the tongue to move forward together with the mandible as well as expands a space in which the tongue is positioned so that the tongue is naturally placed in the anterior region of the palate to induce conversion of mouth breathing to nasal breathing, thereby improving both snoring and sleep apnea.

## BRIEF DESCRIPTION OF THE DRAWINGS

FIG. 1 is a view illustrating an oral appliance according to an embodiment of the present disclosure and a user's dentition in an oral cavity.

FIG. 2 is a front perspective view of an oral appliance according to an embodiment of the present disclosure.

FIG. 3 is a bottom perspective view of an oral appliance according to an embodiment of the present disclosure.

FIG. 4 is a view illustrating a state in which an oral appliance is worn according to an embodiment of the present disclosure.

FIG. 5 is a view illustrating a flow of air in a human body when a user breathes, without wearing the oral appliance according to an embodiment of the present disclosure.

FIG. 6 is a view illustrating a flow of air in a human body when a user wears an oral appliance according to an embodiment of the present disclosure breathes.

FIG. 7 is a front perspective view of an oral appliance according to an embodiment of the present disclosure.

FIG. 8 is a top view of an oral appliance according to an embodiment of the present disclosure.

FIG. 9 is a side perspective view of an oral appliance according to an embodiment of the present disclosure.

FIG. 10 is a cross-sectional view taken along line A-A' of FIG. 11 of an oral appliance according to an embodiment of the present disclosure.

FIG. 11 is a view illustrating a state in which an oral appliance according to an embodiment of the present disclosure is worn only on a dentition of a maxilla as viewed from the inside of an oral cavity.

FIG. 12 is a view illustrating a flow of air in a human body during mouth breathing.

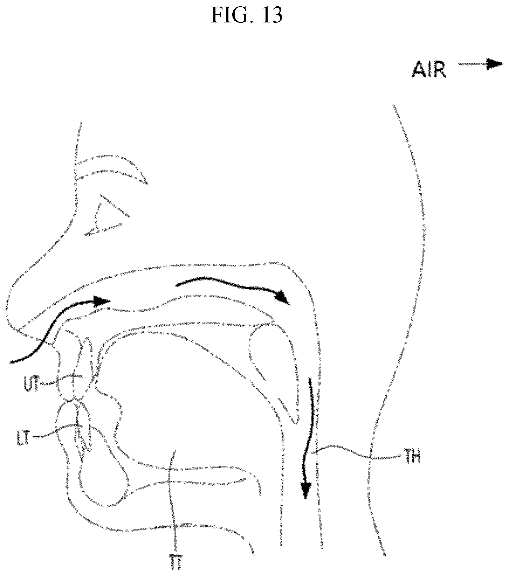

FIG. 13 is a view illustrating a flow of air in a human body during nasal breathing.

FIG. 14 is a view illustrating a breathing state in the case of using an oral appliance according to an embodiment of the present disclosure.

FIG. 15 is a CT image obtained by imaging the user's side before an oral appliance is mounted.

FIG. 16 is a CT image obtained by imaging the user's side after an oral appliance according to an embodiment of the present disclosure is mounted.

## DESCRIPTION OF EXEMPLARY EMBODIMENTS

Hereinafter, embodiments of the present disclosure will be described in detail with reference to the accompanying drawings so that those skilled in the art may easily carry out the present disclosure.

As described above, in the existing maxilla and mandible separated oral appliance, the mandible may move during sleep, but still the movement of the mandible is constrained by the connection member, causing the mandibular joint or surrounding muscles or ligaments to become stiff or painful. In addition, in the existing maxilla and mandible integrated oral appliance, the mandible moves forward by fixing the posterior region, so that the temporomandibular joint and surrounding muscles are stressed and the ligaments are stiff during sleep for a long time. Therefore, it is difficult to wear the oral appliance for a long time and the oral appliance causes pain to the user. In addition, as the mandible moves forward, the tongue also moves forward and protrudes, and the tongue protruding forward retreats backward during long-term sleep. As a result, the airway located at the back of the tongue is pressed, thereby reducing the effect of alleviating snoring and sleep apnea. In addition, when snoring and sleep apnea occur, an oxygen deficiency gradually worsens and the human body performs mouth breathing by opening the mouth to increase the insufficient amount of oxygen. When the mouth is open for mouth breathing, the mandible rotates backwards and downwards and the tongue associated with the mandible also loses contact with the roof of the mouth and is positioned at a lower portion, and the tongue lowered downwards gradually hangs back over time to press the airway to aggravate snoring and sleep apnea.

Therefore, as a solution, the present disclosure provides an oral appliance detachably worn in an oral cavity, the oral appliance having a shape corresponding to a dentition in the oral cavity, including a first dentition accommodation portion formed in a shape corresponding to a dentition of a maxilla and accommodating the dentition of the maxilla, a second dentition accommodation portion formed in a shape corresponding to a dentition of a mandible and accommodating the dentition of the mandible, and a spacer connecting the first dentition accommodation portion and the second dentition accommodation portion and formed to have a predetermined thickness so that the dentition of the maxilla and the dentition of the mandible are spaced apart from each other by a predetermined interval or greater.

Accordingly, the present disclosure may relieve the stiffness of the temporomandibular joint and surrounding muscles by allowing the mandible to move forward naturally, without fixing the posterior region. Therefore, the present disclosure has the effect of improving the user's wearing comfort and remarkably relieving pain when worn for a long time during sleep, because there is no strain on the temporomandibular joint and surrounding muscles. In addition, the present disclosure may alleviate the phenomenon that the tongue excessively presses the airway by allowing the tongue to move forward together with the mandible as well as expands a space in which the tongue is positioned and to be naturally located in the anterior region of the palate. Accordingly, the present disclosure may induce a natural conversion of mouth breathing to nasal breathing, thereby improving snoring and sleep apnea, and at the same time, alleviating side effects on a gnathology system.

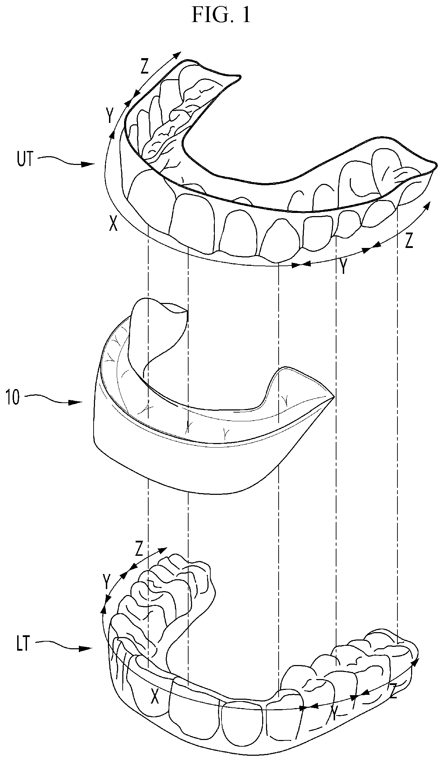

FIG. 1 is a view illustrating an oral appliance and the user's oral dentition according to an embodiment of the present disclosure. Referring to FIG. 1, the user's dentition in the oral cavity includes an anterior region X, a premolar region Y, and a molar region Z including incisors and canines. The oral appliance 10 is formed in a shape corresponding to the dentition of the anterior region X among them. Specifically, the oral appliance 10 is formed in a shape corresponding to the dentition of the anterior region X in the dentition of the maxilla UJ and a shape corresponding to the dentition of the anterior region X in the dentition of the mandible LJ and accommodates the anterior region X of the dentition of the user's oral cavity but does not accommodate the posterior region Z.

As described above, the present disclosure does not fix the molar region, so that the mandible may move forward naturally and relieve stiffness of the temporomandibular joint and surrounding muscles. Accordingly, the temporomandibular joint and surrounding muscles are not strained, and thus, there is an effect of not causing pain to the user even when worn for a long time during sleep. As a result, the wearing comfort is significantly improved, so that it is easy to put on for a long time during sleep to effectively improve snoring.

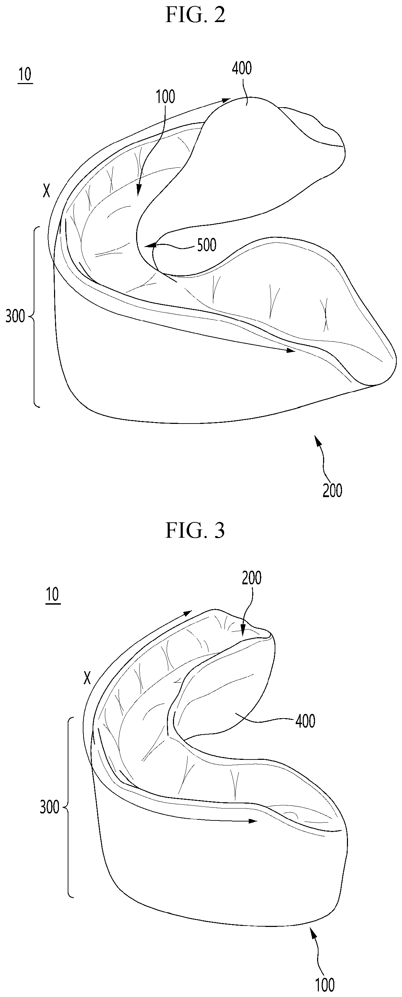

FIG. 2 is a front perspective view of an oral appliance according to an embodiment of the present disclosure, and FIG. 3 is a bottom perspective view of an oral appliance according to an embodiment of the present disclosure. Referring to FIG. 2 and Fig. 3, the oral appliance 10 is formed in a shape corresponding to the dentition of the anterior region in the oral cavity. In addition, the oral appliance 10 includes the first dentition accommodation portion 100, a second dentition accommodation portion 200, and a spacer 300 connecting the first dentition accommodation portion 100 and the second dentition accommodation portion 200. The dentition accommodation portions 100 and 200 are formed to accommodate the anterior region X corresponding to the incisors and canines in the oral cavity of the human body.

Accordingly, by accommodating the anterior region X, the oral appliance 10 of the present disclosure allows a user's mandible to move forward effectively to expand the airway to prevent snoring and sleep apnea and induce air entering the mouth to enter the nose.

First, the oral appliance 10 is inserted into the oral cavity to be worn detachably and is formed in a shape corresponding to the dentition in the oral cavity.

The oral appliance 10 has a shape corresponding to the dentition in the oral cavity as shown in FIGS. 1-3. Specifically, an upper portion of the first dentition accommodation portion 100 corresponds to an upper set of teeth (UT) of an maxilla UJ, which is the 'maxilla', and a lower portion of the second dentition accommodation portion 200 is formed in a shape corresponding to a lower set of teeth (LT) of a mandible LJ, which is the 'mandible'.

In addition, the present disclosure may be a maxilla and mandible integrated oral appliance 10 and may be integrally formed without a separate connection member. That is, the first dentition accommodation portion 100, the second dentition accommodation portion 200, and the spacer 300 described above may be integrally formed without a separate connection member.

In the case of the maxilla and mandible separated oral appliance connected by the connection member, the movement of the mandible is constrained, so that the mandibular joint, surrounding muscles, ligaments, etc. are stiff, and thus, pain easily occurs. In the present disclosure, the maxilla and mandible integrated oral appliance are provided, the oral appliance allows physiological movement in the oral cavity when worn, thereby solving the problems mentioned above.

A material constituting the oral appliance 10 is a material that may be commonly used in the art, and any material may be used without limitations as long as it is not harmful to the human body when the user wears the appliance in the oral cavity and forms the configuration of the present disclosure. For example, a medical resin (a dental resin) or a silicone material may be used.

Meanwhile, the method of forming the oral appliance 10 may be a method commonly used in the art, and any method may be used without limitations as long as the method is appropriate for the material in use. Preferably, after making a real or virtual model of the user's teeth and surrounding tissues, it is possible to use a method of forming an actual or virtual model of the user's teeth or surrounding tissues and molding the same or to use a method of manufacturing by a 3D printer or CAD/CAM milling machine after 3D design.

Next, the first dentition accommodation portion 100 formed in a shape corresponding to the dentition UT of the maxilla UJ and accommodating the dentition UT of the maxilla UJ and the second dentition accommodation portion 200 formed in a shape corresponding to the dentition LT of the mandible LJ and accommodating the dentition LT of the mandible LJ will be described.

As shown in FIG. 2, the first dentition accommodation portion 100 is formed at an upper portion of the oral appliance 100. The first dentition accommodation portion 100 is formed in a shape corresponding to the dentition UT of the maxilla UJ and functions to accommodate the teeth of the maxilla UJ. In addition, the second dentition accommodation portion 200 is formed in a shape corresponding to the dentition LT of the mandible LJ and functions to accommodate the teeth of the mandible LJ.

To this end, an upper recess 200 and a lower recess 300 as a whole have a shape corresponding to the dentition UT of the maxilla UJ and a shape corresponding to the dentition LT of the mandible LJ and may have a horseshoe shape. Also, in some cases, the upper recess 200 and a lower recess 300 may have a chord shape and may have a shape corresponding to each tooth included in the dentitions UT and LT of the maxilla UJ and the mandible LJ.

The method of forming the first dentition accommodation portion 100 and the second dentition accommodation portion 200 at the upper and lower portions of the oral appliance 10 is not particularly limited, and a model of the user's teeth may be first made and molded to correspond thereto or a teeth model file may be designed and worked and formed by a 3D printer or CAD/CAM milling machine. That is, any known method may be used.

In addition, in the existing maxilla and mandible integrated oral appliance, the mandible moves forward by accommodating the anterior region and the posterior region, so that the temporomandibular joint and surrounding muscles are stressed and the ligaments are stiff during sleep for a long time. Therefore, it is difficult to wear the oral appliance for a long time and the oral appliance causes pain to the user.

Through this, the present disclosure accommodates the anterior region of the user's teeth and does not accommodate the posterior region, so that the temporomandibular joint and surrounding muscles are not strained, thereby not causing pain to the user even when worn for a long time during sleep. As a result, the wearing comfort may be significantly improved, since it is easy to put on for a long time during sleep, snoring may be effectively improved.

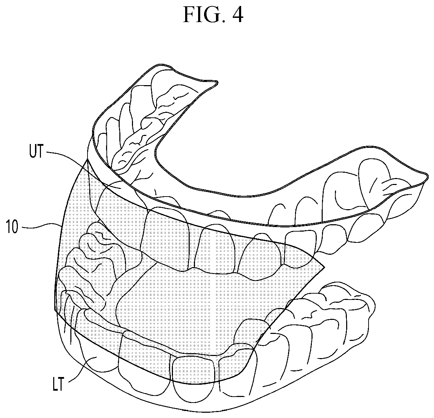

FIG. 4 is a view showing a state in which an oral appliance according to an embodiment of the present disclosure is worn. Referring to FIG. 4, the oral appliance 10 accommodates only the anterior region X in the dentitions UT and LT of the maxilla UJ and the mandible LJ so that the mandible naturally moves forward. In addition, it can be seen that the oral appliance 10 does not fix the premolar region Y and the molar region Z, thereby minimizing stress applied to the temporomandibular j oint. That is, the oral appliance 10 of the present disclosure may be maintained in a comfortable state for the user's gnathology system even when the user wears the oral appliance 10 for a long time.

Meanwhile, when the anterior region of the mandible LJ is accommodated in the second dentition accommodation portion 200, the mandible LJ may move forward at a predetermined distance. In this case, when the mandible LJ moves forward, a phenomenon in which the tongue droops backward, when the user lies down, to directly press the airway or to press the uvula to press the airway as the uvula is pressed backward is alleviated to allow air to flow smoothly, thereby preventing snoring and sleep apnea.

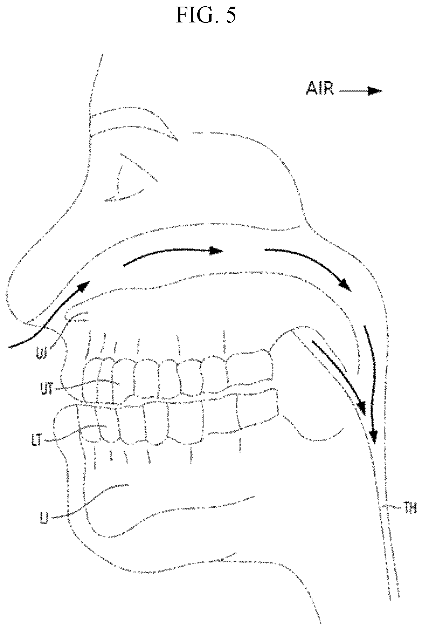

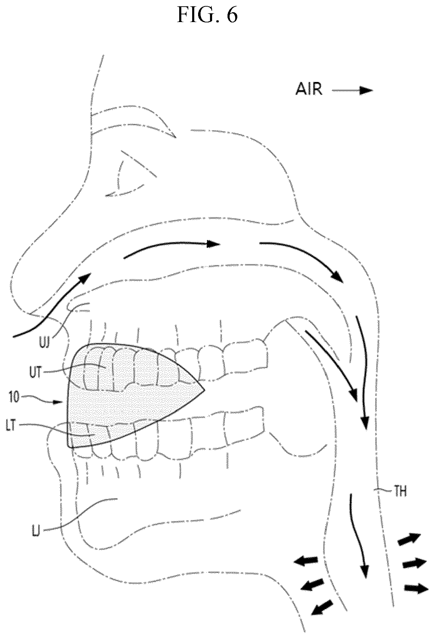

In this regard, FIG. 5 is a view illustrating a flow of air in a human body when a user breathes, without wearing the oral appliance 10 according to an embodiment of the present disclosure, and FIG. 6 is a view illustrating a flow of air in a human body when the user wears the oral appliance 10 according to an embodiment of the present disclosure breathes.

Referring to FIG. 5, in the case of a person who snores, when air inhaled through the nose passes through the nasal passages and flows to the uvula or the back of the tongue, the airway TH of the corresponding section narrows and the air flow is stagnated. Here, the surrounding soft tissues vibrate to cause snoring. Also, when the air flow itself is stopped, sleep apnea occurs.

Referring to FIG. 6, when the user wears the oral appliance 10 of the present disclosure, the user's state may be checked. When the oral appliance 10 of the present disclosure is worn, the user's mandible LJ moves forward and the tongue connected to the mandible LJ also moves forward by a predetermined amount, thereby reducing pressure on the airway TH. Accordingly, air flows smoothly, thereby preventing snoring and sleep apnea. In addition, it may help prevent or treat rhinitis or sinusitis and improve mouth breathing into nasal breathing.

According to an embodiment of the present disclosure, the second dentition accommodation portion 200 may be positioned ahead of the first dentition accommodation portion by a predetermined distance so that the teeth of the mandible LJ may move forward rather than a natural position when the anterior region of the mandible LJ is accommodated therein. That is, referring to FIG. 4, the first dentition accommodation portion 100 and the second dentition accommodation portion 200 may be formed so that the anterior region of the mandible LJ is positioned ahead of the anterior region of the maxilla UJ by a predetermined distance.

According to an embodiment of the present disclosure, the first dentition accommodation portion 100 and the second dentition accommodation portion 200 may fix the user's mandible LJ by adjusting a distance between the inner wall surface of the oral appliance 10 adjacent to the oral cavity and the anterior region of the maxilla UJ or the anterior region of the mandible LJ. For example, since the oral appliance 10 is fixed to the mandible LJ, the second dentition accommodation portion 200 may be formed so that there is no gap between the inner wall surface of the oral appliance 10 adjacent to the oral cavity and the teeth of the mandible LJ and the first dentition accommodation portion 100 may be formed so that the inner wall surface of the oral appliance 10 adjacent to the oral cavity is spaced apart from the teeth by a predetermined distance.

That is, according to the present disclosure, the mandible LJ may be fixed by adjusting a thickness of the recesses of the first dentition accommodation portion 100 and the second dentition accommodation portion 200 according to the user's dentition and dental conditions. Specifically, the oral appliance 10 of the present disclosure fixes the user's mandible LJ by adjusting the interval between the inner wall surfaces of the first dentition accommodation portion 100 and the second dentition accommodation portion 200 adjacent to the oral cavity but does not accommodate the molar region, so that the mandible LJ may be fixed not to stiffen the mandibular LJ joint, surrounding muscles or ligaments.

In addition, the inner wall of the second dentition accommodation portion 200 adjacent to the oral cavity is formed to be fixed by frictional contact with the user's teeth, and the first dentition accommodation portion 100 may be formed to be spaced apart from the teeth of the maxilla UJ by a predetermined distance in a vertical direction and horizontal direction.

That is, as described above, the second dentition accommodation portion 200 may be formed to have a shape corresponding to the dentition LT of the mandible LJ and may be formed so as not to have a gap with the teeth. Accordingly, the anterior region of the mandible LJ may be closely fitted to the second dentition accommodation portion 200, so that the teeth of the mandible LJ may be fixed to the second dentition accommodation portion 200 by frictional contact. In addition, unless an external force greater than the frictional force between the second dentition accommodation portion 200 and the teeth of the mandible LJ is applied, the teeth of the mandible LJ are not easily separated from the second dentition accommodation portion 200, thereby prevent snoring and sleep apnea even when the oral appliance 10 is worn for a long time.

Meanwhile, the first dentition accommodation portion 100 may be formed to be spaced apart from the teeth of the maxilla UJ by a predetermined distance in the vertical and horizontal directions. Due to the vertical and horizontal interval between the first dentition accommodation portion 100 and the anterior region of the maxilla UJ, the teeth of the maxilla UJ may be easily released from the first dentition accommodation portion 100 when necessary, so that the jaw may move physiologically move while in use.

Accordingly, since the user may move the jaw naturally, it is possible to resolve stiffness of the surrounding muscles and ligaments, while effectively fixing the user's mandible LJ, in a state in which the mandible LJ is moved forward.

In addition, a range of the interval at which the first dentition accommodation portion 100 is spaced apart from the teeth of the maxilla UJ in the vertical or horizontal direction may be added to or subtracted from a reference value so as to be applied without limitations according to a tilt angle of an overall occlusal surface according to the user, protrusion of the teeth, a tilt state of the teeth forward, backward, inward, and outward, or a position of the teeth deviating from the overall dentition.

Preferably, the interval at which the first dentition accommodation portion 100 is vertically spaced apart from the teeth of the maxilla UJ may be determined by the user's dentition, a state of teeth, etc., and the interval between the teeth of the maxilla UJ and the teeth of the mandible LJ may be determined by the above interval and a natural interval between the teeth of the maxilla UJ and the teeth of the mandible LJ.

Meanwhile, according to an embodiment of the present disclosure, at least a portion of the inner wall between the first dentition accommodation portion 100 and the second dentition accommodation portion 200 may be open. Through this, when the user wears the oral appliance 10, it is possible to minimize a discomfort caused as a lower surface of the teeth (a surface that touches when chewing food using the teeth) is closed.

Preferably, the inner wall corresponding to the incisors between the first dentition accommodation portion 100 and the second dentition accommodation portion 200 may be open. Specifically, a portion corresponding to a lower surface of the incisors in the inner wall between the first dentition accommodation portion 100 and the second dentition accommodation portion 200 may be open. Through this, even when the oral appliance 10 is worn for a long time, the discomfort that the user may feel may be minimized.

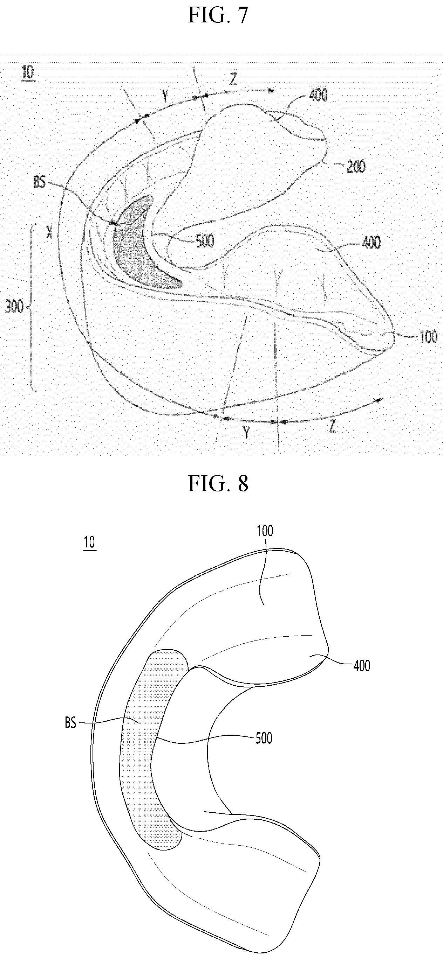

In this regard, FIG. 7 is a front perspective view of the oral appliance 10 according to an embodiment of the present disclosure. Referring to FIG. 7, it can be seen that the inner wall between the first dentition accommodation portion 100 and the second dentition accommodation portion 200 of the oral appliance 10 is partially open (BS).

In addition, FIG. 8 is a top view of the oral appliance 10 according to an embodiment of the present disclosure. Referring to FIG. 8, it can be seen that the open portion BS is the inner wall between the first dentition accommodation portion 100 and the second dentition accommodation portion 200 of the portion corresponding to the user's incisors when the user wears the oral appliance 10.

According to an embodiment of the present disclosure, the oral appliance 10 may further include a spacer connecting the first dentition accommodation portion 100 and the second dentition accommodation portion 200 and having a predetermined thickness so that the dentition of the maxilla UJ and the dentition of the mandible LJ are spaced apart by a predetermined distance or greater.

The spacer 300 may be formed to have a predetermined thickness so that the first dentition accommodation portion 100 and the second dentition accommodation portion 200 are spaced apart from each other by a predetermined distance or greater. Accordingly, the dentition UT of the maxilla UJ and the dentition LT of the mandible LJ may be spaced apart from each other by a predetermined interval, so that the maxilla and the mandible may be open by the thickness of the spacer, thereby expanding space in the oral cavity.

Accordingly, discomfort caused as the tongue is unnaturally fixed or mounted in the oral cavity may be prevented, and the user's tongue may be comfortably mounted for a long time. As a result, the user's gnathology system may be maintained to be comfortable.

Here, the 'thickness' used in the present disclosure refers to a thickness in a direction perpendicular to a horizontal plane based on a direction and angle in which the oral appliance 10 is worn in the user's oral cavity.

The thickness of the spacer 300 may be within a thickness range in which the first dentition accommodation portion 100 and the second dentition accommodation portion 200 are spaced apart from each other by a predetermined interval, but may preferably satisfy following equation 1.

[Equation 1]

$$\frac{T_1 + T_2}{6} \leq Ts \leq T_1 + T_2$$

Here, $T_1$ is a thickness of the first dentition accommodation portion 100, $T_2$ is a thickness of the second dentition accommodation portion 200, and $T_S$ is a thickness of the spacer 300.

More preferably, the thickness of the spacer 300 may satisfy $\frac{T_1 + T_2}{4} \leq Ts \leq T_1 + T_2$ .

When the thickness of the spacer 300 satisfies the above range, the dentition UT of the maxilla UJ and the dentition LT of the mandible LJ may be spaced apart by a predetermined distance or greater, when worn, to expand the space in the oral cavity and prevent the maxilla UJ and mandible LJ from being open too wide apart to put a strain on the temporomandibular joint.

If the thickness of the spacer 300 is less than the above range, the dentition UT of the maxilla UJ and the dentition LT of the mandible LJ are not sufficiently spaced apart from each other and a sufficient space in the oral cavity cannot be secured. In this case, the tongue may be unnaturally fixed or mounted in the oral cavity. In addition, if the thickness of the spacer 300 exceeds the above range, the maxilla UJ and the mandible LJ may be open too wide apart, which may cause a side effect of putting strain on the temporomandibular joint.

Meanwhile, the oral appliance 10 according to the present disclosure may further include a guide portion formed at both end portions of the first dentition accommodation portion guiding the user's teeth to be accommodated in the first dentition accommodation portion 100 and the second dentition accommodation portion 200 as the inner wall surface of the first dentition accommodation portion adjacent to the oral cavity extends by a predetermined distance.

Referring to FIG 2, the guide portion 400 may be formed by extending the first dentition accommodation portion 100 of the oral appliance 10 and may extend below the first dentition accommodation portion 100. Through this, the guide portion 400 may guide the user's teeth to be easily accommodated in the first dentition accommodation portion 100 and the second dentition accommodation portion 200 when worn by the user.

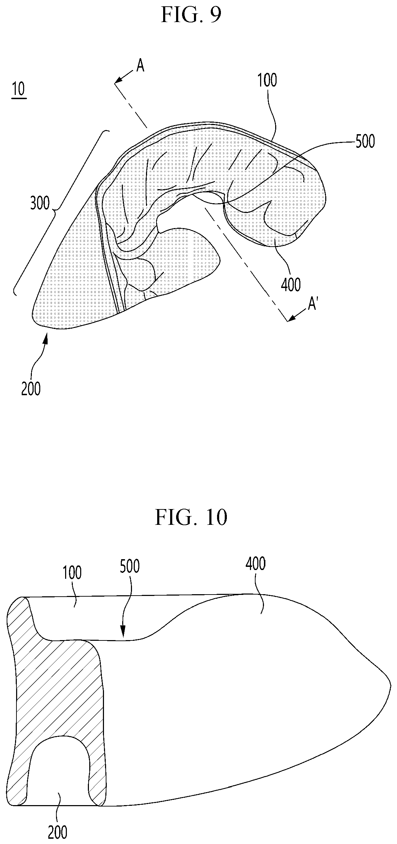

Specifically, FIG. 9 is a side perspective view of the oral appliance 10 according to an embodiment of the present disclosure, and FIG. 10 is a cross-sectional view of the oral appliance 10 according to an embodiment of the present disclosure, taken along line A-A'. Referring to FIG. 9, it can be seen that the guide portion 400 is formed at both ends of the first dentition accommodation portion 100. In addition, referring to FIG. 10, it can be seen that the inner wall surface of the first dentition accommodation portion 100 of the oral appliance 10 adjacent to the oral cavity extends by a predetermined distance to form the guide portion 400.

In this way, when the guide portion 400 is formed by extending the first dentition accommodation portion 100 of the oral appliance 10, the guide portion 400 may also perform a function of forming the first dentition accommodation portion 100. Specifically, the present disclosure has a structure in which the inner wall surface corresponding to the incisors adjacent to the oral cavity is open in the first dentition accommodation portion 100 by forming the tongue tip mounting portion 500 as will be described later, and the teeth of the maxilla UJ may be accommodated through the guide portion, and the guide portion may also perform a function of guiding the teeth of the maxilla UJ and the mandible LJ to be easily accommodated in the first dentition accommodation portion 100 and the second dentition accommodation portion 200.

If the guide portion 400 is formed in the second dentition accommodation portion 200, the oral appliance 10 may be formed structurally such that the mandible moves forward by fixing the maxilla UJ through the first dentition accommodation portion 100. In the case of forming the oral appliance 10 to fix the maxilla UJ in this manner, it may be difficult to form the tongue tip mounting portion 500 having a structure in which the inner wall surface corresponding to the incisors adjacent to the oral cavity is open in the first dentition accommodation portion 100. Without the tongue tip mounting portion 500, when the oral appliance 10 is worn for a long time, the tongue protruding forward may retreat backward during long-term sleep, and as a result, the airway located at the back of the tongue may be pressed, degrading the effect of alleviating snoring and sleep apnea, and mouth breathing continues.

In the meantime, the guide portion 400 may be formed in a position corresponding to the dentition of the canine of the first dentition accommodation portion 100. Preferably, the guide portion 400 may extend in a longitudinal direction along the dentition of the canine in the anterior region of the maxilla UJ to further accommodate at least a portion of the premolar portion of the maxilla UJ.

In this regard, referring to FIG. 1, it can be seen that the first dentition accommodation portion 100 accommodates the anterior region X but does not accommodate the molar region Z and may further accommodate a portion of the premolar region Y as the guide portion 400 is formed in a position corresponding to the dentition of the canine and extends along the dentition. Meanwhile, it can be seen that the second dentition accommodation portion 200 accommodates only the anterior region X and does not accommodate the premolar region Y and the molar region Z.

In this regard, referring to FIG. 1, it can be seen that the first dentition accommodation portion 100 accommodates the anterior region X but does not accommodate the molar region Z and may further accommodate a portion of the premolar region Y as the guide portion 400 is formed in a position corresponding to the teeth of the canine and extends along the dentition. Meanwhile, it can be seen that the second dentition accommodation portion 200 accommodates only the anterior region X and does not accommodate the premolar region Y and the molar region Z.

Referring to FIG. 4, it can be seen that, as the guide portion 400 extends along the dentition from the first dentition accommodation portion 100 in a longitudinal direction, the oral appliance 10 further accommodates a portion of the premolar region Y of the maxilla UJ.

A shape of the guide portion 400 is not particularly limited, and a shape capable of performing the functions described above may be appropriately adopted in a range that does not cause discomfort to the user's oral movement.

In addition, the guide portion 400 may be formed on the right or left of an upper portion of the first dentition accommodation portion 100, which may be determined in consideration of the user's oral structure or oral convenience. Preferably, the guide portion 400 may be formed in a shape in which the right and left sides of the oral appliance 10 correspond to each other. In this case, it is easier to accommodate the teeth of the maxilla UJ and it is possible to solve the problem that the oral appliance 10 is too easily detached even when worn by the user.

Next, the tongue tip mounting portion 500, which is a space in which the tongue tip may be mounted as the inner wall surface corresponding to the incisors in the anterior region of the first dentition accommodation portion 100, adjacent to the oral cavity, is open, will be described.

Through the tongue tip mounting portion 500, the user may easily place the tongue behind the incisors in the front of the palate and the anterior region of the maxilla UJ when wearing the oral appliance 10. Accordingly, the tongue may be mounted foremost within the physiological range and an airway expansion effect may be maximized, so that a phenomenon in which the airway positioned at the rear of the tongue is pressed may be alleviated, thereby preventing snoring and sleep apnea.

Referring to FIGS. 9 and 10, it can be seen that the tongue tip mounting portion 500 is formed as the inner wall surface corresponding to the incisors of the first dentition accommodation portion 100, adjacent to the oral cavity, is open. In this manner, since the tongue tip mounting portion 500 is formed in a structure in which the front is open, a space for the tip of the tongue to be mounted may be provided, and even when the oral appliance 10 is worn, the tongue may be placed in a natural position on a front upper side.

According to an embodiment of the present disclosure, since the tongue tip mounting portion 500 does not have an inner wall surface corresponding to the incisors in the anterior region of the first dentition accommodation portion 100, adjacent to the oral cavity, the tongue tip of the user may be mounted on the rear of the upper incisors, when the user wears the oral appliance 10. Therefore, even when the oral appliance is worn, if the tip of the tongue may be naturally mounted on the back of the upper incisors, it will be possible to provide the user with comfort even when the oral appliance is worn.

The present disclosure has the tongue tip mounting portion 500, so that when a user wears the oral appliance 10 according to the present disclosure, the tip of the tongue may be naturally mounted on the back of the upper incisors, so that there is no discomfort or foreign body feeling for a long time during sleep.

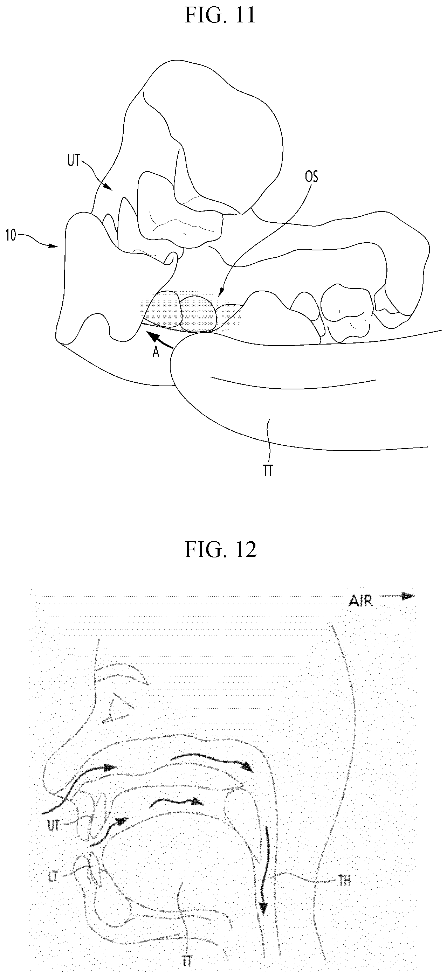

FIG. 11 is a view illustrating a state in which the oral appliance 10 according to an embodiment of the present disclosure is worn only on the dentition UT of the maxilla UJ as viewed from the inside of an oral cavity. Referring to FIG. 11, when the user wears the oral appliance 10, the user's tongue TT may be naturally mounted on the back side OS of the upper incisors in an A direction. Accordingly, the tongue may be mounted foremost within the physiological range.

Meanwhile, FIG. 11 shows a state in which the oral appliance 10 is worn only on the dentition UT of the maxilla UJ in order to help understanding on the present disclosure, but the wearing state of the oral appliance 10 of the present disclosure is not limited thereto.

The present disclosure has the effect of inducing nasal breathing by including the tongue tip mounting portion 500.

In general, when snoring and sleep apnea occur, oxygen deficiency occurs, and the human body performs mouth breathing in which the mouth is open to increase the insufficient amount of oxygen. When the mouth is open for mouth breathing, the mandible LJ rotates backwards and downwards, and accordingly, the tongue TT, which associates with the mandible LJ, is separated from the roof of the mouth and lowered, and as time goes by, the tongue TT, which is gradually lowered downward, sags backwards, causing pressure on the airway. As a result, snoring and sleep apnea worsen.

Specifically, FIG. 11 is a view illustrating a flow of air in a human body during mouth breathing. Referring to FIG. 11, as the mandible LJ rotates backward and downward during mouth breathing, the tongue TT connected to the mandible LJ is also lowered. As described above, the downwardly lowered tongue TT droops back and presses the airway, thereby causing snoring and sleep apnea.

Meanwhile, FIG. 12 is a view illustrating a flow of air in a human body during nasal breathing. Referring to FIG. 12, since the mandible LJ and the tongue TT do not move downward during nasal breathing, a phenomenon in which the downwardly lowered tongue TT droops backwards to press the airway does not occur. Therefore, in order to effectively prevent snoring and sleep apnea, it is necessary to avoid mouth breathing and induce nasal breathing.

Accordingly, in the present disclosure, the tongue tip mounting portion 500 is provided so that the user avoids mouth breathing and smoothly performs nasal breathing, while using the oral appliance 10. Specifically, the tongue is guided to be mounted on the anterior region of the palate by the tongue tip mounting portion 500. In this manner, air sucked into the mouth is physically blocked by the tongue mounted on the anterior region of the palate during mouth breathing, and mouth breathing may be converted into nasal breathing naturally.

As nasal breathing increases, a phenomenon in which the tongue droops down to excessively press the airways is eliminated and air flows more smoothly into the airway, thereby effectively preventing snoring and sleep apnea. In addition, swelling of the tonsils and dryness of the mouth are reduced and the amount of air inhaled through the nose increases, increasing the amount of lung activity, thereby improving the quality of breathing and improving oxygen saturation.

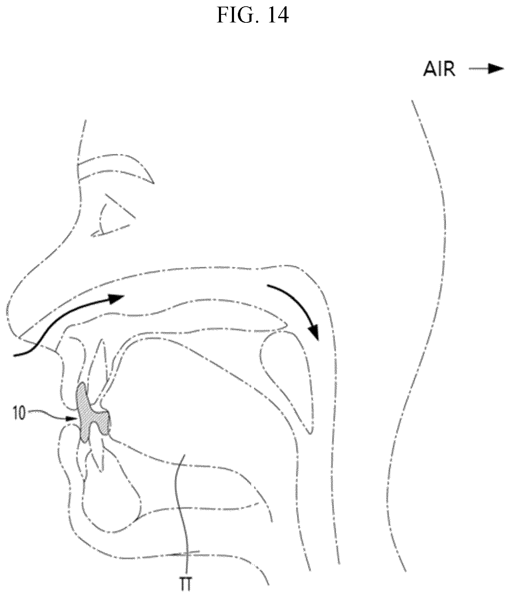

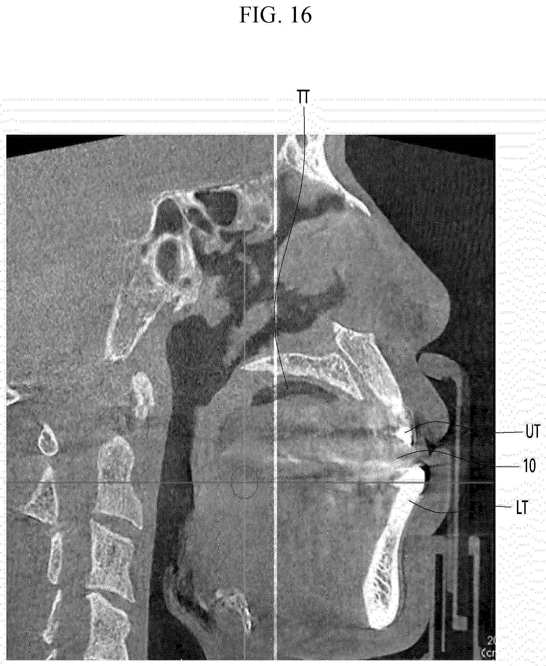

FIG. 14 is a view illustrating a breathing state in the case of using an oral appliance 10 according to an embodiment of the present disclosure. Referring to FIG. 16, when the oral appliance 10 is worn, the user mainly performs nasal breathing (A) instead of mouth breathing (B). The user's tongue TT is stably mounted on the anterior region of the palate by the tongue tip mounting portion 500, and accordingly, air inhaled into the mouth is physically blocked, so mouth breathing (B) is naturally converted into nasal breathing (A).

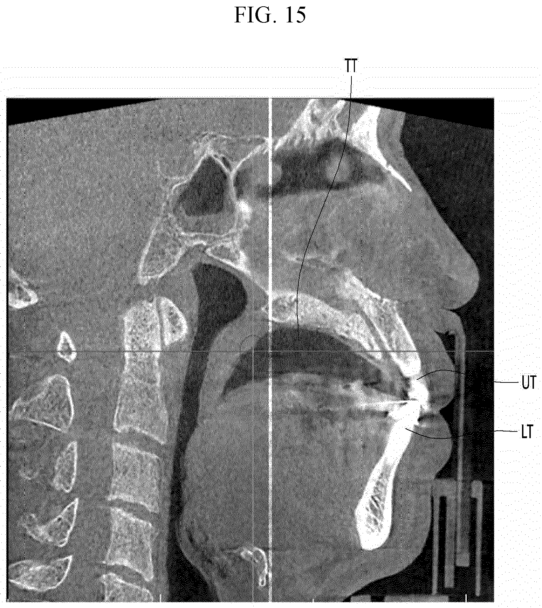

In addition, FIG. 15 is a CT image obtained by imaging the user's side before the oral appliance 10 is mounted, and FIG. 16 is a CT image obtained by imaging the user's side after the oral appliance 10 according to an embodiment of the present disclosure is mounted. Referring to FIGS. 15 and 16, it can be seen that the tongue TT may be naturally mounted on the back of the upper incisors in the oral cavity even after the oral appliance 10 is worn. Accordingly, in the present disclosure, discomfort caused by the tongue being unnaturally fixed or mounted in the oral cavity may be prevented by expanding the space in the oral cavity and the user's tongue may be comfortably mounted for a long time.

That is, the tongue tip mounting portion 500 of the present disclosure allows the user's tongue TT to be mounted on the back of the upper incisors to provide comfort when worn, so that the tongue TT may be positioned stably in the anterior region of the palate, alleviating symptoms such as pressing the airway or the like and inducing conversion of mouth breathing to nasal breathing, thereby effectively preventing snoring and sleep apnea.

As a result, the use of the oral appliance 10 of the present disclosure may effectively treat snoring and sleep apnea. In addition, by accommodating the anterior region but not the posterior region, natural movement may be supported even when the oral appliance 10 is used for a long time, and accordingly, the stiffness of the related muscles and ligaments caused by the movement of the mandible LJ forward may be alleviated. In addition, even when the oral appliance 10 is worn, pain, malocclusion, or temporomandibular joint disorder may not occur, and rhinitis or sinusitis may be improved by allowing air to flow smoothly through the airway.

The oral appliance 10 described as described above is not that the configuration of the embodiment described above may be limitedly applied, but all or part of each embodiment may be selectively combined so that various modifications may be made.

## Claims

1. An oral appliance detachably worn in an oral cavity, the oral appliance having a shape corresponding to a dentition of an anterior region in the oral cavity, the oral appliance comprising:

   a first dentition accommodation portion (100) formed in a shape corresponding to the dentition of the anterior region of a maxilla and accommodating the anterior region of the maxilla;
   a second dentition accommodation portion (200) formed in a shape corresponding to the dentition of the anterior region of a mandible and accommodating the anterior of the mandible;
   a spacer (300) connecting the first dentition accommodation portion (100) and the second dentition accommodation portion (200) and formed to have a predetermined thickness so that the dentition of the maxilla and the dentition of the mandible are spaced apart from each other by a predetermined interval or greater, **characterized in that** the oral appliance further comprises:

   a tongue tip mounting portion (500) as a space in which an inner wall surface adjacent to the oral cavity corresponding to incisors of the first dentition accommodation portion (100) is open to allow the tip of the tongue to be mounted, and
   a guide portion (400) formed at both end portions of the first dentition accommodation portion (100) guiding the user's teeth to be accommodated in the first dentition accommodation portion (100) and the second dentition accommodation portion (200) as the inner wall surface of the first dentition accommodation portion (100) adjacent to the oral cavity extends by a predetermined distance,
   wherein the first dentition accommodation portion (100), the second dentition accommodation portion (200), and the spacer (300) are integrally formed,
   wherein the guide portion (400) is formed in a position corresponding to the dentition of a canine of the first dentition accommodation portion (100),
wherein the dentition of the maxilla and the dentition of the mandible are spaced apart from each other by a predetermined interval such that the maxilla and the mandible are open by the predetermined thickness of the spacer (300), thereby expanding space in the oral cavity,
the thickness of the spacer (300) satisfies Equation 1 below:

[Equation 1]

$$\frac{T_1 + T_2}{4} \leq Ts \leq T_1 + T_2$$

wherein $T_1$ is a thickness of the first dentition accommodation portion (100), $T_2$ is a thickness of the second dentition accommodation portion (200), and $T_S$ is a thickness of the spacer (300).

2. The oral appliance of claim 1, wherein the tongue tip mounting portion (500) does not have an inner wall surface adjacent to the oral cavity corresponding to the incisors of the first dentition accommodation portion (100) so that the tip of the user's tongue is placed behind upper incisors when worn by the user.

3. The oral appliance of claim 1, wherein the guide portion (400) extends in a longitudinal direction along the dentition of the canine in the anterior region of the maxilla to further accommodate at least a portion of the premolar portion of the maxilla.

4. The oral appliance of claim 1, wherein an inner wall of the second dentition accommodation portion (200) adjacent to the oral cavity is formed so that the user's teeth are fixed by frictional contact, and the first dentition accommodation portion (100) is formed to be spaced apart from the teeth of the maxilla by a predetermined distance in vertical and horizontal directions.

5. The oral appliance of claim 1, wherein at least a portion of an inner wall between the first dentition accommodation portion (100) and the second dentition accommodation portion (200) is open.

6. The oral appliance of claim 5, wherein the inner wall between the first dentition accommodation portion (100) and the second dentition accommodation portion (200) corresponds to an inner wall of a portion corresponding to the incisors.

**Patentansprüche**

1. Abnehmbar in einer Mundhöhle getragene orale Vorrichtung, wobei die orale Vorrichtung eine Form aufweist, die einem Gebiss eines vorderen Bereichs in der Mundhöhle entspricht, wobei die orale Vorrichtung Folgendes umfasst:

einen ersten Gebissaufnahmeabschnitt (100), der in einer Form ausgebildet ist, die dem Gebiss des vorderen Bereichs eines Oberkiefers entspricht und den vorderen Bereich des Oberkiefers aufnimmt;
einen zweiten Gebissaufnahmeabschnitt (200), der in einer Form ausgebildet ist, die dem Gebiss des vorderen Bereichs eines Unterkiefers entspricht und den vorderen Bereich des Unterkiefers aufnimmt;
einen Abstandshalter (300), der den ersten Gebissaufnahmeabschnitt (100) und den zweiten Gebissaufnahmeabschnitt (200) verbindet und so ausgebildet ist, dass er eine vorbestimmte Dicke aufweist, so dass das Gebiss des Oberkiefers und das Gebiss des Unterkiefers um ein vorbestimmtes oder größeres Intervall voneinander beabstandet sind, **dadurch gekennzeichnet, dass** die orale Vorrichtung ferner Folgendes umfasst:

einen Zungenspitzenbefestigungsabschnitt (500) als Raum, in dem eine an die Mundhöhle angrenzende Innenwandfläche, die den Schneidezähnen des ersten Gebissaufnahmeabschnitts (100) entspricht, offen ist, um die Befestigung der Zungenspitze zu ermöglichen, und
einen Führungsabschnitt (400), der an beiden Endabschnitten des ersten Gebissaufnahmeabschnitts (100) ausgebildet ist und die Zähne des Benutzers führt, die in dem ersten Gebissaufnahmeabschnitt (100) und dem zweiten Gebissaufnahmeabschnitt (200) aufgenommen werden sollen, während sich die Innenwandfläche des ersten an die Mundhöhle angrenzenden Gebissaufnahmeabschnitts (100) um einen vorbestimmten Abstand erstreckt,
wobei der erste Gebissaufnahmeabschnitt (100), der zweite Gebissaufnahmeabschnitt (200) und der Abstandshalter (300) einstückig ausgebildet sind,
wobei der Führungsabschnitt (400) in einer Position ausgebildet ist, die dem Gebiss eines Eckzahns des ersten Gebissaufnahmeabschnitts (100) entspricht,
wobei das Gebiss des Oberkiefers und das Gebiss des Unterkiefers um ein vorbestimmtes Intervall voneinander beabstandet sind, so dass der Oberkiefer und der Unterkiefer um die vorbestimmte Dicke des Abstandshalters (300) geöffnet sind, wodurch sich der Raum in der Mundhöhle ausdehnt, wobei die Dicke des Abstandshalters (300) die folgende Gleichung 1 erfüllt:

[Gleichung 1]

$$\frac{T_1 + T_2}{4} \leq Ts \leq T_1 + T_2$$

wobei $T_1$ eine Dicke des ersten Gebissaufnahmeabschnitts (100) ist, $T_2$ eine Dicke des zweiten Gebissaufnahmeabschnitts (200) ist und $T_s$ eine Dicke des Abstandshalters (300) ist.

2. Orale Vorrichtung nach Anspruch 1, wobei der Zungenspitzenbefestigungsabschnitt (500) keine an die Mundhöhle angrenzende Innenwandfläche aufweist, die den Schneidezähnen des ersten Gebissaufnahmeabschnitts (100) entspricht, so dass die Zungenspitze des Benutzers beim Tragen durch den Benutzer hinter den oberen Schneidezähnen angeordnet wird.

3. Orale Vorrichtung nach Anspruch 1, wobei sich der Führungsabschnitt (400) in Längsrichtung entlang des Gebisses des Eckzahns in dem vorderen Bereich des Oberkiefers erstreckt, um ferner zumindest einen Abschnitt des Prämolarenabschnitts des Oberkiefers aufzunehmen.

4. Orale Vorrichtung nach Anspruch 1, wobei eine Innenwand des zweiten an die Mundhöhle angrenzenden Gebissaufnahmeabschnitts (200) so ausgebildet ist, dass die Zähne des Benutzers durch Reibungskontakt fixiert werden, und der erste Gebissaufnahmeabschnitt (100) so ausgebildet ist, dass er um einen vorbestimmten Abstand in vertikaler und horizontaler Richtung von den Zähnen des Oberkiefers beabstandet ist.

5. Orale Vorrichtung nach Anspruch 1, wobei mindestens ein Abschnitt einer Innenwand zwischen dem ersten Gebissaufnahmeabschnitt (100) und dem zweiten Gebissaufnahmeabschnitt (200) offen ist.

6. Orale Vorrichtung nach Anspruch 5, wobei die Innenwand zwischen dem ersten Gebissaufnahmeabschnitt (100) und dem zweiten Gebissaufnahmeabschnitt (200) einer Innenwand eines Abschnitts entspricht, der den Schneidezähnen entspricht.

**Revendications**

1. Appareil buccal porté de manière amovible dans une cavité buccale, l'appareil buccal ayant une forme correspondant à une dentition d'une région antérieure dans la cavité buccale, l'appareil buccal comprenant :

une première partie de logement de la dentition (100) formée selon une forme correspondant à la dentition de la région antérieure d'un maxillaire et logeant la région antérieure du maxillaire ;
une deuxième partie de logement de la dentition (200) formée selon une forme correspondant à la dentition de la région antérieure d'une mandibule et logeant la partie antérieure de la mandibule ;
un espaceur (300) reliant la première partie de logement de la dentition (100) et la deuxième partie de logement de la dentition (200) et formé pour avoir une épaisseur prédéterminée de sorte que la dentition du maxillaire et la dentition de la mandibule soient espacées l'une de l'autre par un intervalle prédéterminé ou supérieur, **caractérisé en ce que** l'appareil buccal comprend en outre :

une partie de montage du bout de la langue (500) sous la forme d'un espace dans lequel une surface de paroi interne adjacente à la cavité buccale correspondant aux incisives de la première partie de logement de la dentition (100) est ouverte pour permettre de monter le bout de la langue, et
une partie de guidage (400) formée aux deux parties d'extrémités de la première partie de logement de la dentition (100) guidant les dents de l'utilisateur pour être logés dans la première partie de logement de la dentition (100) et la deuxième partie de logement de la dentition (200) lorsque la surface de la paroi interne de la première partie de logement de la dentition (100) adjacente à la cavité buccale s'étend sur une distance prédéterminée,

où la première partie de logement de la dentition (100), la deuxième partie de logement de la dentition (200) et l'espaceur (300) sont formés d'un seul tenant,
où la partie de guidage (400) est formée dans une position correspondant à la dentition d'une canine de la première partie de logement de la dentition (100),
où la dentition du maxillaire et la dentition de la mandibule sont espacées l'une de l'autre par un intervalle prédéterminé de telle sorte que le maxillaire et la mandibule sont ouverts par l'épaisseur prédéterminée de l'espaceur (300), élargissant ainsi l'espace dans la cavité buccale,

l'épaisseur de l'espaceur (300) satisfait à l'équation 1 ci-dessous :

[Équation 1]

$$\frac{T_1 + T_2}{4} \leq Ts \leq T_1 + T_2$$

où $T_1$ est l'épaisseur de la première partie de logement de la dentition (100), $T_2$ est l'épaisseur de la deuxième partie de logement de la dentition (200) et Ts est l'épaisseur de l'espaceur (300).

2. Appareil buccal selon la revendication 1, où la partie de montage du bout de la langue (500) ne présente pas de surface de paroi interne adjacente à la cavité buccale correspondant aux incisives de la première partie de logement de la dentition (100), de sorte que le bout de la langue de l'utilisateur soit placé derrière les incisives supérieures lorsque l'utilisateur porte l'appareil.

3. Appareil buccal selon la revendication 1, où la partie de guidage (400) s'étend dans une direction longitudinale le long de la dentition de la canine dans la région antérieure du maxillaire afin de loger en outre au moins une partie de la partie prémolaire du maxillaire.

4. Appareil buccal selon la revendication 1, où une paroi interne de la deuxième partie de logement de la dentition (200) adjacente à la cavité buccale est formée de sorte que les dents de l'utilisateur soient fixées par contact par frottement, et la première partie de logement de la dentition (100) est formée pour être espacée des dents du maxillaire par une distance prédéterminée dans les directions verticale et horizontale.

5. Appareil buccal selon la revendication 1, où au moins une partie d'une paroi interne entre la première partie de logement de la dentition (100) et la deuxième partie de logement de la dentition (200) est ouverte.

6. Appareil buccal selon la revendication 5, où la paroi interne entre la première partie de logement de la dentition (100) et la deuxième partie de logement de la dentition (200) correspond à une paroi interne d'une partie correspondant aux incisives.

FIG. 1

FIG. 2

FIG. 3

FIG. 4

FIG. 5

FIG. 6

## FIG. 7

## FIG. 8

## FIG. 9

## FIG. 10

## FIG. 11

## FIG. 12

FIG. 13

AIR →

UT

LT

TT

TH

FIG. 14

FIG. 15

## FIG. 16

**EP 4 173 601 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101463021